# FASCICULE DE BREVET EUROPEEN

(11) **EP 1 888 767 B1**
(45) Date de publication et mention de la délivrance du brevet: **30.08.2017**
(21) Numéro de dépôt: 06794431.4
(22) Date de dépôt: 16.05.2006
(51) Int. Cl.: C12Q 1/04

(54) **MILIEU REACTIONNEL POUR LES BACTERIES VIBRIO**
REAKTIONSMEDIUM FÜR VIBRIO-BAKTERIEN
REACTION MEDIUM FOR VIBRIO BACTERIA

(30) Priorité: 18.05.2005 FR 0551283
(43) Date de publication de la demande: 20.02.2008
(73) Titulaire: BIOMERIEUX, 69280 Marcy-L'Etoile (FR)
(72) Inventeur: MONGET, Daniel, F-01150 Saint-Sorlin-en-Bugey (FR); ROBICHON, Denis, F-01150 Blyes (FR)
(74) Mandataire: Bitaud, Valérie Marie-Odile
(86) Numéro de dépôt international: PCT/FR2006/050446
(87) Numéro de publication internationale: WO 2007/000530

(56) Documents cités:
- WO-A-02/00922
- US-A- 4 308 346
- MIYAMOTO T. ET AL.: "Improved fluorogenic assay for rapid detection of Vibrio parahaemolyticus in foods." APPL. ENVIRON. MICROBIOL., vol. 56, no. 5, mai 1990 (1990-05), pages 1480-1484, XP002365823
- CHATTERJEE B.D. ET AL.: "Studies on the beta-D-galactosidase activity of Vibrio parahaemolyticus." INDIAN J. MED. RES., vol. 60, 6 juin 1972 (1972-06-06), pages 831-833, XP001003086
- KAPER J.B. ET AL.: "Cholera" CLIN. MICROBIOL. REV., vol. 8, no. 1, janvier 1995 (1995-01), pages 48-86, XP002365824
- AMARO C. ET AL.: "Vibrio vulnificus biotype 2, pathogenic for eels, is also an opportunistic pathogen for humans." APPL. ENVIRON. MICROBIOL., vol. 62, no. 4, avril 1996 (1996-04), pages 1454-1457, XP002365825
- DONOVAN T.J. ET AL.: "Culture media for the isolation and enumeration of pathogenic Vibrio species in foods and environmental samples." INT. J. FOOD MICROBIOL., vol. 26, 1995, pages 77-91, XP002365826
- EDDABRA R. ET AL.: "Evaluation of a new chromogenic medium, chromID(TM) Vibrio, for the isolation and presumptive identification ofandfrom human clinical specimens", EUR. J. CLIN. MICROBIOL. INFECT. DIS., vol. 30, no. 6, June 2011 (2011-06), pages 733-737, XP019899370,

## Description

La présente invention concerne un milieu de culture pour la détection des bactéries Vibrio. L'invention concerne également l'utilisation de ce milieu, ainsi qu'une méthode pour identifier des bactéries Vibrio.

Le choléra est une maladie diarrhéique très contagieuse, due à un bacille à Gram négatif, *Vibrio cholerae.* De même, *Vibrio parahaemolyticus* est responsable de gastro-entérites aiguës. Les souches bactériennes responsables de ces pathologies sont transmises par voie orale à partir d'eau ou d'aliments contaminés. En raison du caractère épidémique de ces maladies, il est fréquemment nécessaire de détecter ces bactéries chez des patients contaminés, ou dans les environnements tels que les aliments et l'eau.

Le milieu de référence actuellement utilisé pour la détection des *Vibrio* est le milieu TCBS (Thiosulfate Citrate Bile salt Saccharose). La gélose TCBS est un milieu sélectif, préconisé pour la recherche et l'isolement des *Vibrio* pathogènes (norme NF ISO 8914 et recommandations Organisation Mondiale de la Santé). La forte concentration en bile et en citrate, associée à un pH élevé (pH = 8,6) permet l'élimination de nombreuses bactéries. La principale source de carbone est le saccharose. L'utilisation du saccharose se traduit par une diminution du pH, et par le virage de l'indicateur de pH du vert au jaune. Les colonies saccharose positives, telles que plusieurs espèces de *Vibrio* notamment *V. cholerae,* apparaissent donc jaunes. Les colonies saccharose négatives, comme *V. parahaemolyticus,* apparaissent vertes. Toutefois, ce milieu n'est pas très sensible, ni très spécifique et de nombreux faux positifs sont détectés (c'est à dire qu'on détecte des bactéries qui sont considérées comme étant des Vibrio alors qu'elles ne le sont pas).

Il existe également le milieu CHROMagar Vibrio. Ce milieu chromogénique sélectif permet la différentiation de *V. parahaemolyticus, V.vulnificus* et *V.cholerae* par rapport aux autres espèces de Vibrio. *V. parahaemolyticus* apparaît sous forme de colonies mauves, *V.vulnificus* et *V.choleare* produisent des colonies bleues tandis que d'autres espèces telle que *V. alginolyticus* forme des colonies incolores. Ce milieu se présente sous forme de poudre. Le principe de ce milieu est basé sur la détection simultanée des activités ß-glucosidase, spécifique de *V. parahaemolyticus,* et ß-galactosidase, spécifique de *V*. *cholerae* et *V*. *vulnificus,* en présence d'une forte concentration en saccharose. Toutefois, ce milieu présente, outre quelques espèces identifiées comme étant de faux positifs, une sensibilité de détection des *V*. *cholerae* moyenne. WO02/00922 divulgue un milieu de culture destiné à mettre en évidence les bactéries du genre *Vibrio'* caractérisé en ce qu'il comporte au moins un agent chromogène choisi parmi les substrats de la β-glucosidase et les substrats de la β-galactosidase. Son utilisation pour la discrimination des bactéries *V. cholerae* de *V. parahaemolyticus* est aussi revendiquée. Ladite méthode est basée sur le fait que *V*. *cholerae* présente une activité β-galactosidase sans présenter d'activité β-glucosidase. Ainsi l'ajout d'un agent chromogène substrat pour la β-galactosidase dans un milieu de culture des *Vibrio* permet de distinguer *V. cholerae* des autres vibrions par coloration des colonies formées.

(Miyamoto T. et al., Appl. Environm. Microbiol., 1990, 56(5): 1480-1484) décrit un test fluorogénique de détection de *V. parahaemolyticus* dont l'amélioration consiste en l'ajout dans le milieu d'arabinose et de glucuronate ce qui permet un enrichissement spécifique en *V. parahaemolyticus* par comparaison aux autres bactéries du genre *Vibrio.*

L'invention se propose de résoudre les lacunes de l'état de la technique en présentant un milieu de culture sensible et spécifique, pour isoler et identifier des *Vibrio* groupe cholerae (*choleraelvulnificus* et *mimicus)* et *Vibrio parahaemolyticus.*
A ce titre l'invention concerne un milieu réactionnel pour les bactéries groupe cholerae (*choleraelvulnificus* et *mimicus)* et *Vibrio parahaemolyticus* comprenant
- un substrat permettant la détection d'une activité enzymatique β-galactosidase
- un sucre qui est le L-arabinose et un sucre qui est le glucuronate
- un indicateur coloré choisi parmi le rouge neutre et le bleu de bromothymol

Au sens de la présente invention, on entend par milieu réactionnel, un milieu comprenant tous les éléments nécessaires à la survie et/ou à la croissance de microorganismes.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.

Le milieu réactionnel peut être solide, semi-solide ou liquide. Par milieu solide, on entend par exemple un milieu gélifié. Préférentiellement, ce milieu est constitué par un milieu gélifié. L'agar est l'agent gélifiant traditionnel en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

Le milieu de culture selon l'invention peut contenir d'éventuels autres additifs comme par exemple : des peptones, un ou plusieurs facteurs de croissance, des hydrates de carbone, un ou plusieurs agents sélectifs, des solutions tampons, un ou plusieurs gélifiants... Ce milieu de culture peut se présenter sous forme de liquide, de gel prêt à l'emploi, c'est à dire prêt à l'ensemencement en tube, flacon, ou sur boite de Petri. Lorsque la présentation est en flacon, on réalise préférentiellement une régénération (passage à 100°C) préalable du milieu avant de couler en boîte de Pétri. Préférentiellement, le milieu selon l'invention est un milieu sélectif, c'est à dire un milieu comprenant des inhibiteurs de croissance des bactéries et levures que l'on ne souhaite pas détecter.

Au sens de la présente invention, le substrat est choisi parmi tout substrat pouvant être hydrolysé en un produit qui permet la détection, directe ou indirecte d'une activité enzymatique β-galactosidase. Préférentiellement, ce substrat comprend
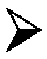 une première partie spécifique de l'activité enzymatique à révéler. Cette première partie est susceptible d'interagir avec ladite enzyme, qui est spécifique du microorganisme recherché,
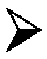 une seconde partie faisant office de marqueur, ci-après appelée partie marqueur, qui peut être fluorescente ou chromogène.

Comme substrat fluorescent, on peut citer notamment les substrats à base d'umbelliférone ou d'aminocoumarine, à base de résorufine ou encore à base de fluorescéïne.

Les substrats enzymatiques de l'invention sont utilisables dans une large gamme de pH, notamment entre pH 5,5 et 10.

La concentration de substrat enzymatique de l'invention dans le milieu réactionnel est comprise entre 0,01 et 2 g/l et, avantageusement, elle est de 0,075 g/l. En effet, à cette concentration de substrat, on obtient un meilleur contraste de coloration.

Selon un mode préféré de réalisation de l'invention, ledit substrat est un substrat chromogène X-β-Galactoside, avec X représentant la partie marqueur. Préférentiellement, le substrat utilisé est le 5-Bromo-4-Choro-3-indoxyl-beta D-galactopyranoside. Préférentiellement, ce substrat est présent dans le milieu à une concentration comprise entre 0,01 et 2 g/l, préférentiellement entre 0,05 et 1,15 g/l.

Selon un autre mode de réalisation de l'invention, le substrat peut être choisi parmi les substrats de β-Galactosidase comme le RedA-β-Galactoside, Magenta-β-Galactoside, GreenA-β-Galactoside, Rose-β-Galactoside et Alizarine-β-Galactoside qui sont sensibles pour la détection de *V*. *cholerae.* Le L-arabinose est présent dans le milieu à une concentration comprise entre 1 et 30 g/l, préférentiellement entre 5 et 15 g/l. Préférentiellement, la concentration est d'environ 8 g/l.

Selon un mode préféré de réalisation de l'invention, l'indicateur coloré est le rouge neutre. Préférentiellement, cet indicateur coloré est présent dans le milieu à une concentration comprise entre 0,0001 et 0,05 g/l, préférentiellement entre 0,002 et 0,010 g/l préférentiellement entre 0,003 et 0,007 g/l. L'indicateur coloré peut être également le bleu de bromothymol. Selon un mode préféré de réalisation de l'invention, la concentration est d'environ 0,010 g/l. Afin d'améliorer la spécificité de détection des *Vibrio parahaemolyticus* et *Vibrio cholerae* par rapport aux autres espèces bactériennes susceptibles d'être présentes dans le milieu, un deuxième sucre qui est le glucuronate est présent dans le milieu à une concentration comprise entre 1 et 30 g/l, préférentiellement entre 5 et 15 g/l. Préférentiellement, la concentration est d'environ 8 g/l.

L'invention concerne également l'utilisation *in vitro* du milieu tel que défini ci dessus pour isoler et identifier des *Vibrio* groupe cholerae (*choleraelvulnificus* et *mimicus)* et *Vibrio parahaemolyticus.*

Lors de l'utilisation de ce milieu, les *Vibrio cholerae* sont détectées par une activité β-Galactosidase spécifique qui permet d'obtenir des colonies bleues à vertes. Les *Vibrio parahaemolyticus* sont mises en évidence par l'utilisation spécifique du L-arabinose qui permet l'obtention de colonies roses (coloration très intense) résultant d'une acidification dans le milieu faisant virer l'indicateur coloré (rouge neutre) de l'incolore au rose vif. En présence de sels biliaires, la coloration reste concentrée au niveau de la colonie, ce qui facilite la lecture des milieux. Les autres espèces de *Vibrio* apparaissent violettes ou incolores.

L'invention concerne enfin une méthode d'identification *in vitro* de bactéries *Vibrio cholerae* et *Vibrio parahaemolyticus* selon laquelle on détecte l'activité beta galactosidase pour identifier *Vibrio cholerae* et l'acidification d'un sucre pour détecter *Vibrio parahaemolyticus.*

Selon un mode préféré de réalisation de l'invention, on détecte l'activité beta-galactosidase pour identifier *Vibrio cholerae* par l'utilisation d'un substrat chomogènique specifique de l'activité beta-galactosidase. Ce substrat est tel que défini précédemment, et est préférentiellement le X-β-Galactoside.

Selon un mode préféré de réalisation de l'invention, on détecte l'acidification d'un sucre par l'utilisation d'un indicateur coloré, préférentiellement le rouge neutre, qui change de couleur lors de la variation de pH induite par l'acidification du sucre.

Les exemples suivants sont donnés à titre illustratif et n'ont aucun caractère limitatif. Ils permettront de mieux comprendre l'invention.

### 1. Choix des souches et des échantillons alimentaires

### a- Evaluation du milieu à partir de souches pures :

Le milieu selon l'invention a été testé sur 69 souches dont 22 souches de *Vibrio* (8 *V.cholerae, 2 V.mimicus,* 2 *V. vulnificus,* 4 *V. parahaemolyticus, 2 V. alginolyticus, 2 V. fluvialis, 1V. hollisae et 1V. metschnikovii) ;* 8 souches de *Staphylococcus ;* 6 souches de *Pseudomonas ;* 6 souches de *Candida ;* 4 souches de *Enterococcus ;* 3 souches de *Enterobacter ;* 2 souches de *Escherichia coli ;* 2 souches de *Klebsiella ;* 2 souches de *Proteus ;* 2 souches de *Salmonella ;* 2 souches de *Shigella;* 2 souches de *Yersinia;* 2 souches de *Aeromonas;* 2 souches de *Moraxella; 2* souches de *Plesiomonas;* 1 souche de *Listeria;* 1 souche de *Acinetobacter*

### b-Evaluation du milieu à partir d'aliments contaminés artificiellement :

Les 4 matrices alimentaires suivantes : Huîtres, Crevettes, Filet de loup surgelé et Truite de mer entière ont été contaminées par *V. cholerae.* Les 5 autres matrices, Filet de julienne, Gambas congelées, Moules, Terrine de poisson et Surimi ont été contaminées par *V. parahaemolyticus.*

Le protocole utilisé est celui décrit par les normes NF ISO 8914 et NF EN ISO 6887-3 préconisant un pré-enrichissement 7 à 8 h à 37°C en Eau Peptonnée Salée (EPS). Les contaminations artificielles sont réalisées à partir d'une suspension bactérienne à 0,5 McFarland diluée à 10⁻⁵. Un volume de 1 ml ou 0,1 ml de cette dilution est utilisé pour contaminer respectivement les poissons/autres crustacés et les coquillages. Cette contamination ayant lieu après le broyage des matrices alimentaires en EPS et stockage 24 h à 2-8 °C mais avant l'incubation de 7-8 h à 37°C. Un échantillon de 10 µl de chaque pré-enrichissement est ensuite étalé sur le milieu selon l'invention qui est incubé 48 h à 37°C. En parallèle, un essai témoin est réalisé dans les mêmes conditions que précédemment mais sans contamination artificielle. Ceci permet de vérifier d'une part, la flore naturelle de chaque matrice, et d'autre part, de s'assurer qu'il n'existe pas d'incompatibilité entre le milieu selon l'invention et la détection des colonies de *Vibrio.*

### 2. Préparation du milieu selon l'invention

Le milieu selon l'invention est un milieu sélectif comprenant comme milieu de base le milieu Trypcase-Soja (bioMérieux réf. 43 011), et comprenant les éléments suivants (g/l)

| | |
|---|---|
| IPTG (IsoPropyl ThioGalactopyranoside) | 0,05 |
| Sels biliaires | 0,6 |
| Rouge neutre | 0,005 |
| L-arabinose | 10 |
| 5-Bromo-4-chloro-3-indoxyl-beta-D-galactopyranoside) | 0,1 |

Un deuxième sucre (glucuronate ; 10 g/l) est également ajouté pour améliorer la spécificité de détection des *Vibrio parahaemolyticus* et *Vibrio cholerae.*

### 3. Ensemencement des milieux à partir de souches pures

Les souches de bactéries et levures, toutes issues de la collection de la Demanderesse, mises en suspension dans de l'eau physiologique, ont été ensemencées pour donner des colonies isolées sur le milieu. Les boîtes ont été incubées à 37 °C pendant 48 heures. Les colonies formées ont été examinées visuellement après 24 et plus de 40 heures d'incubation. L'intensité de coloration de ces colonies a été évaluée et chaque souche a ensuite été classée positive ou négative (exprimant ou n'exprimant pas le phénotype recherché) sachant qu'une souche est considérée comme positive pour des valeurs d'intensité de coloration > à 0,5.

Selon le principe du milieu, les souches de *Vibrio cholerae,* exprimant une activité β-Galactosidase (utilisation du X-β-Galactoside), doivent produire des colonies bleu-vertes tandis que celles de *V*. *parahaemolyticus,* utilisant le L-arabinose avec acidification du milieu et virage de l'indicateur coloré, doivent donner des colonies de couleur rose vif. Les autres genres ou espèces peuvent donner des colonies violettes (utilisation de l'arabinose associé à une activité β-Galactosidase) ou incolores (arabinose (-) et β-Galactosidase(-)).

### 4. Lecture de milieux :

### Echelle de lecture des intensités de coloration :

C'est une échelle arbitraire commune à l'ensemble des échantillons biologiques et des milieux testés. Cette échelle est valable pour cette expérience ainsi que pour toutes les expériences qui suivront. Elle peut être définie de la manière suivante :
- 0 correspond à une absence d'activité
- 0,1 correspond à la présence d'une trace de coloration
- 0,5 correspond à la présence d'une coloration très pâle
- 1 correspond à la présence d'une coloration nette de faible intensité
- 2 correspond à la présence d'une coloration franche d'intensité moyenne
- 3 correspond à la présence d'une coloration intense
- 4 correspond à la présence d'une coloration très intense

### a) sur souches pures

Les résultats sont exprimés en % de bon diagnostic par rapport à tous les tests en termes de sensibilité et spécificité après 24 heures d'incubation et sont donnés dans le tableau ci-après, le % de sensibilité correspondant au nombre de vrais positifs détectés sur le milieu par le nombre total de vrais positifs à détecter et le % de spécificité correspondant au nombre de vrais négatifs détectés sur le milieu par le nombre total de vrais négatifs à détecter.

| Sensibilité | | Spécificité | |
|---|---|---|---|
| *V. cholerae* | *V.parahaemolyticus* | *V. cholerae* | *V. parahaemolyticus* |
| 100% | 100% | 100% | 95,5 |

Ces résultats montrent, d'une part, que l'ensemble des souches de *Vibrio cholerae* donnent bien des colonies de couleur attendue (sensibilité 100%) et que, d'autre part, aucune des 57 souches non *V. cholerae* ne développe la couleur caractéristique des *V. cholerae* (spécificité 100%). Les 4 souches de *V. parahaemolyticus* donnent elles aussi des colonies ayant la couleur attendue (sensibilité 100%). Parmi les 65 souches non *V. parahaemolyticus,* seules 3 d'entres elles ont été classées comme étant faux positifs (2/2 *Vibrio fluvialis* et 1/2 *Enterobacter aerogenes)* et génèrent des colonies roses caractéristiques de *V. parahaemolyticus* (spécificité 95,5%).

### b) sur matrices alimentaires

Les résultats présentés dans le tableau ci-après sont ceux observés après 24 h d'incubation à 37°C. Chaque milieu obtenu à partir d'une matrice contaminée artificiellement est comparé au milieu contrôle (obtenu à partir d'une matrice alimentaire non contaminée artificiellement) de sorte à repérer, parmi la flore associée, l'espèce bactérienne théoriquement utilisée pour contaminer l'aliment. Chaque colonie ainsi repérée est alors isolée puis identifiée à l'aide des tests classiques. Si l'identification est confirmée la colonie est alors considérée Vrai +, dans le cas contraire c'est un Faux+. Par ailleurs, en plus des colonies de couleur attendue, une identification est réalisée sur les autres colonies non attendues mais présentant une coloration caractéristique. Si l'identification confirme une espèce appartenant à une autre espèce que celle attendue mais faisant partie du groupe *Vibrio cholerae (vulnificus, mimicus)* ou *parahaemolyticus* il s'agit alors de Vrai +, naturellement présent dans l'aliment, en revanche si l'identification ne confirme pas *Vibrio cholerae (vulnificus, mimicus)* ou *parahaemolyticus,* l'espèce est considérée comme Faux+.

| Sensibilité (nombre de Vrai+ obtenus sur nombre de Vrai+ attendus) | | Spécificité (nombre de Faux+ obtenus) | Lisibilité |
|---|---|---|---|
| *V. cholerae* | *V. parahaemolyticus* | | |
| 4/4 | 5/5 | 4 à 5 Faux + | +++ |

Toutes les souches utilisées pour contaminer les matrices alimentaires ont été retrouvées après pré-enrichissement et isolement sur le milieu selon l'invention. La lisibilité du milieu est tout à fait satisfaisante dans la mesure où il est aisé de repérer les colonies positives, bleu-vertes ou roses, au milieu de la flore annexe même si cette dernière est présente en abondance.

### 5. Avantages du milieu Vibrio ID :

Le milieu Vibrio ID possède d'excellentes performances en terme de sensibilité et spécificité. La lisibilité, c'est à dire la capacité à détecter des colonies positives ou à écarter des colonies négatives, est également très bonne. La sensibilité est très bonne dans le cas d'échantillons faiblement contaminés.

## Revendications

1. Milieu réactionnel pour les bactéries *Vibrio* groupe *cholerae (cholerae*/*vulnificus* et *mimicus*) et *Vibrio parahaemolyticus* comprenant
• un substrat permettant la détection d'une activité enzymatique β-galactosidase
• un sucre qui est le L-arabinose
• un sucre qui est le glucuronate
• un indicateur coloré choisi parmi le rouge neutre et le bleu de bromothymol

2. Milieu réactionnel selon la revendication 1 selon lequel ledit substrat est un substrat chromogène X-β-Galactoside.

3. Milieu réactionnel selon l'une quelconque des revendications 1 ou 2 selon lequel l'indicateur coloré est le rouge neutre.

4. Méthode *d'identification in vitro* de bactéries *Vibrio cholerae* et *Vibrio parahaemolyticus* selon laquelle on détecte l'activité beta-galactosidase pour identifier *Vibrio cholerae* et l'acidification du L-arabinose pour détecter *Vibrio parahemolyticus,* comprenant l'utilisation d'un milieu réactionnel comprenant
• un substrat permettant la détection d'une activité enzymatique β-galactosidase
• un sucre qui est le L-arabinose
• un sucre qui est le glucuronate
• un indicateur coloré choisi parmi le rouge neutre et le bleu de bromothymol

5. Utilisation *in vitro* d'un milieu selon l'une quelconque des revendications 1 à 3 pour isoler et identifier des *Vibrio* groupe *cholerae* (*cholerae*/*vulnificus* et *mimicus)* et *Vibrio parahaemolyticus.*

## Patentansprüche

1. Reaktionsmedium für *Vibrio*-Bakterien der *cholerae-*Gruppe (*cholerae*/*vulnificus* und *mimicus*) und *Vibrio parahaemolyticus*, umfassend
• ein Substrat, das den Nachweis einer β-Galactosidase-Enzymaktivität gestattet
• einen Zucker, bei dem es sich um L-Arabinose handelt
• einen Zucker, bei dem es sich um Glucuronat handelt
• einen Farbindikator, ausgewählt aus Neutralrot und Bromthymolblau.

2. Reaktionsmedium nach Anspruch 1, wobei das Substrat ein chromogenes X-β-Galactosid-Substrat ist.

3. Reaktionsmedium nach einem der Ansprüche 1 oder 2, wobei der Farbindikator Neutralrot ist.

4. *In vitro*-Identifizierungsverfahren für *Vibrio cholerae-* und *Vibrio parahaemolyticus*-Bakterien, wobei man die beta-Galactosidase-Aktivität zur Identifizierung von *Vibrio cholerae* und die Ansäuerung von L-Arabinose zur Identifizierung von *Vibrio parahemolyticus* nachweist, umfassend die Verwendung eines Reaktionsmediums, das Folgendes umfasst:
• ein Substrat, das den Nachweis einer β-Galactosidase-Enzymaktivität gestattet
• einen Zucker, bei dem es sich um L-Arabinose handelt
• einen Zucker, bei dem es sich um Glucuronat handelt
• einen Farbindikator, ausgewählt aus Neutralrot und Bromthymolblau.

5. *In vitro*-Verwendung eines Mediums nach einem der Ansprüche 1 bis 3 zur Isolation und Identifizierung von *Vibrio*-Bakterien der *cholerae*-Gruppe (*cholerae*/*vulnificus* und *mimicus*) und *Vibrio. parahaemolyticus.*

## Claims

1. Reaction medium for *cholerae*-group *Vibrio* (*cholerae*/*vulnificus* and *mimicus)* and *Vibrio parahaemolyticus* bacteria, comprising
• a substrate for detecting a β-galactosidase enzymatic activity
• a sugar which is L-arabinose
• a sugar which is glucuronate.
• a coloured indicator chosen among neutral red and bromothymol blue

2. Reaction medium according to Claim 1, according to which said substrate is an X-β-galactoside chromogenic substrate.

3. Reaction medium according to any one of Claims 1 or 2, according to which the coloured indicator is neutral red.

4. Method for identifying *Vibrio cholerae* and *Vibrio parahaemolyticus bacteria, in vitro,* according to which beta-galactosidase activity is detected for identifying *Vibrio cholerae* and the L-arabinose acidification is detected for revealing *Vibrio parahemolyticus,* comprising the use of a reaction medium comprising:
• a substrate for detecting a β-galactosidase enzymatic activity
• a sugar which is L-arabinose
• a sugar which is glucuronate.
• a coloured indicator chosen among neutral red and bromothymol blue

5. *In vitro* use of a medium according to any one of Claims 1 to 3, for isolating and identifying *cholerae-group Vibrio* (*cholerae*/*vulnificus* and *mimicus)* and *Vibrio parahaemol yticus.*
